# EUROPEAN PATENT SPECIFICATION

(11) **EP 1 609 857 B1**
(45) Date of publication and mention of the grant of the patent: **20.10.2010**
(21) Application number: 04717878.5
(22) Date of filing: 05.03.2004
(51) Int. Cl.: C12N 15/09, A01K 67/027, C12N 1/15, C12N 1/19, C12N 1/21, C12N 5/00

(54) **METHOD OF DEMETHYLATING TARGET METHYLATED REGION OF GENOMIC DNA**
VERFAHREN ZUR DEMETHYLIERUNG EINER METHYLIERTEN ZIELREGION GENOMISCHER DNA
PROCEDE DE DEMETHYLATION D'UNE REGION METHYLEE CIBLE D'ADN GENOMIQUE

(30) Priority: 06.03.2003 JP 2003060615
(43) Date of publication of application: 28.12.2005
(73) Proprietor: TOUDAI TLO, LTD., Tokyo 113-0033 (JP)
(72) Inventor: SHIOTA, Kunio, Chiba 2701423 (JP); TANAKA, Satoshi, Saitama 3510114 (JP); OHGANE, Jun, Tokyo 173-0004 (JP); IMAMURA, Takuya, Saitama 3510114 (JP); HATTORI, Naka, Tokyo 1600003 (JP); NISHINO, Koichiro, Tokyo 1250033 (JP); HATTORI, Naoko, Tokyo 1600003 (JP); YAMAMOTO, Soshi, Tokyo 1940003 (JP)
(74) Representative: Bohnenberger, Johannes
(86) International application number: PCT/JP2004/002914
(87) International publication number: WO 2004/078971

(56) References cited:
- WO-A-00/51424
- SLEUTELS FRANK ET AL: "The non-coding Air RNA is required for silencing autosomal imprinted genes" NATURE (LONDON), vol. 415, no. 6873, 14 February 2002 (2002-02-14), pages 810-813, XP002378944 ISSN: 0028-0836
- WEISS A. ET AL: 'DNA demethylation in vitro: involvement of RNA' CELL vol. 86, no. 5, 06 September 1996, pages 709 - 718, XP002097749
- JOST. JP. ET AL: 'A re-investigation of the ribonuclease sensitivity of a DNA demethylation reaction in chicken embryo and G8 mouse myoblasts' FEBS LETTERS vol. 449, no. 2-3, 23 April 1999, pages 251 - 254, XP004259570
- JOST J-P. ET AL: 'The RNA moiety of chick embryo 5-methylcytosine-DNA glycosylase targets DNA demethylation' NUCLEIC ACIDS RESEARCH vol. 25, no. 22, 1997, pages 4545 - 4550, XP002979070
- VAIRAPANDI M. ET AL: 'Human DNA-demethylating activity: a glycosylase associated with RNA and PCNA' J. OF CELLULAR BIOCHEMISTRY vol. 79, 2000, pages 249 - 260, XP002976904
- IMAMURA T. ET AL: "Non-coding RNA directed DNA demethylation of Sphk1 CpG island" BIOCHEMICAL AND BIOPHYSICAL RESEARCH COMMUNICATIONS, vol. 322, no. 2, pages 593-600, XP004527661 ORLANDO, FL, US

### SEQUENCE LISTING

<110> SHI0TA, Kunio
   <110> TANAKA, Satoshi
   <110> OHGANE, Jun
   <110> IMAMURA, Takuya
   <110> HATTORI, Naka
   <110> NISHINO, Koichiro
   <110> HATTORI, Naoko
   <110> YAMAMOTO, Soshi
<120> Method for demethylating target methylated region of genome DNA
<130> 04717878.5
<160> 2
<210> 1
   <211> 213
   <212> RNA
   <213> Artificial Sequence
<220>
   <223> Description of Artificial Sequence: RNA-A
<400> 1
   cggggcugga acuucucaca cucugcgggc gagcgugggg caaaguugac uggaaacuca 60
   auccgugaga aagagcagag cagcccacag ggcauuuuau acuugagccu cggggccggu 120
   ucccuuucug cucggaagag acccaguggg agggggguuc cagucuggcc cugguuccag 180
   uccgcacaau ggcuccucug uguccgcggc cgc 213
<210> 2
   <211> 927
   <212> RNA
   <213> Artificial Sequence
<220>
   <223> Description of Artificial Sequence: RNA-B
<400> 2
   acccaccucu cuccuugcau cgcuucuuaa aguccugaag aggugagucc gggaggagag 60
   cggacccaga uaagaaagcg uugcagugaa cuaggucacu ccuugaaggu ggcuucucuc 120
   ccagaggacc cuggaguucc ccgaacgagu uucucacuuu auuaggaguc cccgggaggc 180
   cggggcgcag gcuggaccug cggcuccgca cgcgucacug caauacaugc cuugguucug 240
   uacugccgcg gggacagccu gaccggauca ugaacgcccc gagugccuuc cgagaaggug 300
   acucagcgac acgcucacgg guggagaauc ucccggaaac aaccaaccaa ccaaaaauau 360
   aaaauaauau aaaaugagga guuggaggga gcaagaacgu caucgcccac ccccgguucc 420
   gcggcgcgug gccaccaaga ucugguccug cauccgcccc cauccccggc uucccgaacc 480
   ucacagccca ggaauccagc gcggacaagg cgagcagcuc ugcagccgca gugccgcggu 540
   cacgcgcugc ggggacgacg cgagccgcgg ugggaggagg gggcucugcg gggaggccag 600
   ucccucuaag gcucgcuccc uugucuaccc ugucccuccc cuucccagag accuaggauc 660
   cagaucuggc guuaguaucu agcagacuca gagaggaggc uauucucccc gagauuuuga 720
   gcuuggaggg ggcuggcagc ccagacugua ggaaacaccc ucuucugcuc ucccacagcu 780
   ugggaaggag ugucuaucaa gcccgaaaag caagucauua gcagcuaaag aggagaaacu 840
   gaggcacggg gaaggaaaga ucaguccgug uguaaauuca cacucauaag gccgaccugg 900
   aacagggucu cgcuuuucuu ugagucu 927

### SEQUENCE LISTING

<110> SHIOTA, Kunio
   <110> TANAKA, Satoshi
   <110> OHGANE, Jun
   <110> IMAMURA, Takuya
   <110> HATTORI, Naka
   <110> NISHINO, Koichiro
   <110> HATTORI, Naoko
   <110> YAMAMOTO, Soshi
<120> Method for demethylating target methylated region of genome DNA
<130> 04717878.5
<160> 2
<210> 1
   <211> 213
   <212> RNA
   <213> Artificial Sequence
<220>
   <223> Description of Artificial Sequence: RNA-A
<400> 1
   cggggcugga acuucucaca cucugcgggc gagcgugggg caaaguugac uggaaacuca 60
   auccgugaga aagagcagag cagcccacag ggcauuuuau acuugagccu cggggccggu 120
   ucccuuucug cucggaagag acccaguggg agggggguuc cagucuggcc cugguuccag 180
   uccgcacaau ggcuccucug uguccgcggc cgc 213
<210> 2
   <211> 927 <212> RNA
   <213> Artificial Sequence
<220>
   <223> Description of Artificial Sequence: RNA-B
<400> 2
   acccaccucu cuccuugcau cgcuucuuaa aguccugaag aggugagucc gggaggagag 60
   cggacccaga uaagaaagcg uugcagugaa cuaggucacu ccuugaaggu ggcuucucuc 120
   ccagaggacc cuggaguucc ccgaacgagu uucucacuuu auuaggaguc cccgggaggc 180
   cggggcgcag gcuggaccug cggcuccgca cgcgucacug caauacaugc cuugguucug 240
   uacugccgcg gggacagccu gaccggauca ugaacgcccc gagugccuuc cgagaaggug 300
   acucagcgac acgcucacgg guggagaauc ucccggaaac aaccaaccaa ccaaaaauau 360
   aaaauaauau aaaaugagga guuggaggga gcaagaacgu caucgcccac ccccgguucc 420
   gcggcgcgug gccaccaaga ucugguccug cauccgcccc cauccccggc uucccgaacc 480
   ucacagccca ggaauccagc gcggacaagg cgagcagcuc ugcagccgca gugccgcggu 540
   cacgcgcugc ggggacgacg cgagccgcgg ugggaggagg gggcucugcg gggaggccag 600
   ucccucuaag gcucgcuccc uugucuaccc ugucccuccc cuucccagag accuaggauc 660
   cagaucuggc guuaguaucu agcagacuca gagaggaggc uauucucccc gagauuuuga 720
   gcuuggaggg ggcuggcagc ccagacugua ggaaacaccc ucuucugcuc ucccacagcu 780
   ugggaaggag ugucuaucaa gcccgaaaag caagucauua gcagcuaaag aggagaaacu 840
   gaggcacggg gaaggaaaga ucaguccgug uguaaauuca cacucauaag gccgaccugg 900
   aacagggucu cgcuuuucuu ugagucu 927

## Description

### TECHNICAL FIELD

The present invention relates to an in-vitro method for demethylating a target methylated region existing in genome DNA of a living cell, a method for producing a living cell, a method for producing a cell nucleus isolated from the living cell, and a method for producing a tissue or organ produced by culturing the living cell.

### BACKGROUND ART

The fact that a variety of cells that configure the bodies of mammals, excluding some exceptions such as immune cells, have entirely the same gene sets has been demonstrated by the birth of cloned animals such as sheep, mice and cows using nuclear implantation technology in which body cell nuclei are implanted into enucleated unfertilized eggs. Consequently, the differences of the individual cells are generated by the differences in the gene sets that are expressed and the gene sets that are not expressed.

It is known that methylation of genome DNA is related to the expression of genes, and in many cases, the methylation of genome DNA controls gene expression. As a transcription control mechanism based on methylation of genome DNA, for example, there has been a report that methyl CpG bound protein (MeCP2), which binds by recognizing methylated cytosine, functions as a suppressor that controls the binding of transcription factors by binding to the control region of the gene (Nan X, et al., Cell, 1999, vol. 88, p.471-481). Moreover, there has been a report that, together with MeCP2 and Sin3, methylated cytosine forms composites with histone deacetylase, and that histone deacetylase is recruited by MeCP2, the chromatin structure of the methylated genome region is condensed, and is stabilized as an inactive heterochromatin (Jones PL, et all, Nature Genetics, 1998, vol. 19, p. 187-191.) Specifically, cytosine methylation and the change of the chromatin structure by histone acetylation are not mutually independent phenomena, but rather control the gene expression of the various loci in the genome while working closely together.

The amount of methylation of genome DNA is lowest in the blastocyst stage, and appears to increase thereafter in conjunction with differentiation in body cells (Jaenisch R, et al., Trends in Genetics, 1997, vol. 13, p. 323-329). Then, it appears possible that the formation of methylation patterns of genome DNA restricts cell and tissue specific gene expression patterns. Consequently, when anomalies are generated in the methylation patterns of genome DNA, the cells and tissues have difficulty maintaining normal function and traits, and there is the risk of generating various kinds of diseases as a result. Specifically, when anomalies are generated in the methylation patterns of genome DNA, there is the risk that functional insufficiency, termination of function, abnormal growth and abnormal cell death, etc. of cells may be induced, impairment of the normal functions of tissues may come about, and a variety of diseases may be generated on an individual level including developmental anomalies, cancer, and lifestyle-related diseases. For example, there has been a report that methylation of cancer control factors is a cause of the development of cancer (Jones PA, et al., Nature Genetics, 2002, vol. 3, p. 415-428), and there has been a report that anomalies of the methylation pattern of genome DNA is a cause of developmental anomalies and postnatal anomalies in cloned animals (Shiota K, et. al., Differentiation, 2002, vol. 69, p. 162-166).

Sleutels Frank et al. "The non-coding Air RNA is required for silencing autosomal imprinted genes" Nature (London), vol. 415, no. 6873, 14 February 2002 (2002-02-14), pages 810-813, XP002378944 ISSN: 0028-0836 discloses a non-coding RNA (Air) which is required for gene imprinting. Further, this document teaches that the Air non-coding RNA overlaps the Igf2r promoter, belonging to an imprinted gene cluster of the protein-coding genes Igf2r, Slc22a2 and Slc22a3.

WO 00/51424 A (PPL Therapeutics Ltd.; Colman, Alan; Schnieke, Angelika, Elisabeth; KI) 8 September 2000 (2000-09-08) discloses the production of genetically modified animals, in which the genetic modifications are engineered in somatic cells cultured in vitro by gene targeting with suitable vectors. The genetically modified cells are used as nuclear donors for nuclear transfer to produce an transgenic animal embryo.

### DISCLOSURE OF THE INVENTION

Methylation of genome DNA is mainly generated by cytosine having a 5'-CG-3' sequence (CpG sequence). CpG islands, which are dense with the CpG sequence, exist in genome DNA of mammals, and tissue-dependently methylated regions (T-DMR) are present in the CpG islands. Then, multiple CpG islands having T-DMR are present in genome DNA.

It appears that if anomalies are generated in the methylation patterns of genome DNA by regions not originally methylated becoming methylated, then the methylation patterns of genome DNA can be normalized by demethylation of the specific methylated region. Then, if the methylation pattern of the genome DNA can be normalized, it may be possible to prevent and cure diseases arising from anomalies of the methylation pattern of genome DNA, to produce cells, tissues and organs that are useful in regeneration therapy, and to make the production of cloned animals by body cell nuclear implantation more effective, etc.

Compounds having a demethylation action, for example, can be used in the demethylation of methylated regions, and 5-azacytidine and 5-azadeoxycytidine, etc., for example, are known as this kind of compound. Nonetheless, these compounds demethylate the entire genome DNA region or a broad range of an unspecified region. Moreover, it appears that demethylation can be attempted by destroying the DNA methyltransferase gene, but in this case as well, the demethylation region carries across a broad range, and it is not possible to demethylate only a specified methylated region.

Thus, an object of the present invention is to provide a demethylation method that can demethylate only a specified methylated region from among multiple methylated regions present in genome DNA without influencing other methylated regions.

In addition, another object of the present invention is to provide a recombinant vector that can be used in the aforementioned demethylation method, and a demethylation kit comprising the recombinant vector.

A further object of the present invention is to provide a living cell in which the aforementioned recombinant vector is introduced, a cell nucleus isolated from the living cell, a tissue or organ produced by culturing the aforementioned living cell, a cloned animal produced using the aforementioned cell nucleus, a method for producing a tissue or organ by culturing the aforementioned living cell, and a method for producing a cloned animal using the aforementioned cell nucleus.

In order to achieve the aforementioned objects, the present invention provides the following demethylation method, method for producing a living cell, method for producing a cell nucleus isolated from the living cell, and method for producing tissue or organ produced by culturing the aforementioned living cell.
(1) An in-vitro method for demethylating a methylated cytosine residue or residues at CG sites in a target methylated region present in either sense or antisense DNA chain in genome DNA of a living cell, the method comprising a step of artificially generating in the living cell the following RNA (a) or (b),
   (a) RNA in a base length of 100 to 2000 comprising a base sequence equivalent to the base sequence of a region comprising all or part of the target methylated region in the DNA chain where the target methylated region is present,
   (b) RNA in a base length of 100 to 2000 comprising a base sequence equivalent to the base sequence of a region comprising a region neighboring the target methylated region in the DNA chain where the target methylated region is present, wherein the region neighboring the target region is positioned at the 5' terminal side or at the 3' terminal side of the target region and the distance between the neighboring region and the target region is a base length of 0 to 1000,
   wherein the target methylated region resides in CpG islands having tissue - dependently methylated regions in the genome DNA.
(2) The method according to the aforementioned (1), further comprising a step of introducing into the living cell a recombinant vector that can express the RNA (a) or (b).
(3) A method for producing a living cell in which a methylated cytosine residue or residues at CG sites in a target methylated region present in either sense or antisense DNA chain in genome DNA is demethylated by the method according to the aforementioned (1) and (2), with the provisio that the living cell is not a human embryonic stem cell or germ cell.
(4) A method for producing a tissue or organ by culturing a living cell produced by a method according to the aforementioned (3).
(5) A method for producing a cell nucleus by isolation from a living cell produced by the method according to the aforementioned (3).

### BRIEF DESCRIPTION OF THE DRAWINGS

Fig. 1 is a drawing indicating a CpG island of a sphingosine kinase gene derived from rats, the positions where the T-DMR and the translation starting point are present, as well as the corresponding positions of the RNA-A and RNA-B;
Fig. 2 is a drawing indicating the methylation status (%) of representative CpG sites (-3229, -3205, -3139, -3086, -3023) before and after artificially generating RNA-A and RNA-B in NRK cells.

### BEST MODE FOR CARRYING OUT THE INVENTION

The present invention will be explained in detail below.

In the present invention, "living cell" means a living cell that is present in the state of being separated from a living body. Consequently, the living cells actually configuring a living body are not included in the "living cells" of the present invention.

In the present invention, the type of organism from which the living cells are derived is not particularly limited, and may be animal, plant or microbe, etc., but preferably the organism is an animal, and more preferable, a mammal. For example, humans, monkeys, cows, sheep, goats, horses, pigs, rabbits, dogs, cats, rats, mice and guinea pigs, etc. may be cited as mammals.

In the present invention, the type of living cell is for example, body cells, germ cells, stem cells or cultured cells of these, etc. may be cited with the proviso that the living cell is not a human embryonic stem cell or germ cell. Concrete examples of body cells include: cells from neural tissue such as brain and spinal marrow; sensory organs such as retina cells and olfactory cells; gastrointestinal organs such as esophagus, stomach, small intestine and colon; respiratory organs such as lung and bronchia; reproductive organs such as testes, ovary, uterus, and placenta; urinary organs such as kidneys and bladder; hemopoietic organs such as bone marrow cells ; muscle tissue such as skeletal muscle, smooth muscle, and heart muscle; skeletal tissue such as osteoblasts and osteoclasts; and living cells separated from a variety of tissues and organs such as skin tissue such as skin and hair root cells, or cultured cells thereof. Ova, sperm and cultured cells thereof may be cited as concrete examples of germ cells; and embryonic-stem cells (ES cells), embryonic germ cells (EG cells), trophoblast stem cells (TS cells), bone marrow stem cells, and neural stem cells may be cited as concrete examples of stem cells with the proviso that the living cell is not a human embryonic stem cell or germ cell.

In the present invention, the methylated region targeted for demethylation is the specified methylated region present in either sense or antisense chains of genome DNA chains in the genome DNA of living cells. There are multiple cell- and tissue-dependently methylated regions (T-DMR) in the sense and antisense chains of the genome DNA of mammals, and cell- and tissue-dependently methylated patterns are formed, and the target methylated region in the present invention is, for example, a specified T-DMR present in either sense or antisense DNA chains among these multiple T-DMR. Which T-DMR are targeted for demethylation in this situation may be suitably selected corresponding to the objectives for demethylation. The base length of the target methylated region is not particularly limited, but normally is a base length of 1 to 10000, preferably 100 to 2000.

In the present invention, when demethylating the target methylated region, the RNA that is artificially generated in the living cell is RNA (a) or (b) below. The RNA that is artificially generated in the living cell may be both RNA (a) and (b) below.
(a) RNA comprising a base sequence equivalent to the base sequence of the region comprising all or part of the target methylated region in the DNA chain where the target methylated region is present; (b) RNA comprising a base sequence equivalent to the base sequence of the region comprising the region neighboring the target methylated region in the DNA chain where the target methylated region is present.

The base length of RNA (a) or (b) is not particularly limited, and may be suitably adjusted corresponding to the base length, etc. of the target methylated region, but normally is a base length of 100 to 5000, preferably a base length of 100 to 2000.

In RNA (a) or (b), the "DNA chain where the target methylated region is present" means either sense or antisense DNA chain in genome DNA; and "DNA chain where the target methylated region is not present" means the other chain. In addition, "base sequence equivalent to" means that DNA and RNA comprise the same base sequence except that the thymine (T) is uracil (U).

In RNA (a), "the region comprising all or part of the target methylated region in the DNA chain where the target methylated region is present" includes the region consisting of all or part of the target methylated region, the region consisting of the 5' terminal side or 3' terminal side of the target methylated region (region positioned on the 5' terminal side or 3' terminal side of the target methylated region) and regions adjacent thereto, and the region consisting all of the target methylated region and the adjacent regions of the 5' terminal side and/or 3' terminal side thereof, etc. Moreover, "part of the target methylated region" includes any part of the target methylated region, is not particularly limited in base length, and may be suitably adjusted corresponding to the base length of the target methylated region, etc., but normally, is a base length of 1 to 1000, preferably a base length of 1 to 200.

In RNA (b) "the region comprising the region neighboring the target methylated region in the DNA chain where the target methylated region is present" includes the region consisting of the region adjacent to the target methylated region, and the region consisting of the region neighboring the target methylated region and the adjacent regions of the 5' terminal side and/or 3' terminal side thereof, etc. Moreover, "the region neighboring the target methylated region" means the region positioned at the 5' terminal side or at the 3' terminal side of the target methylated region, and the distance to the target methylated region is normally a base length of 0 to 1000, preferable a base length of 0 to 100. Here, "the distance between the neighboring region and the target methylated region" means the distance from the 3' terminal of the target methylated region to the 5' terminal of the neighboring region, or the distance from the 5' terminal of the target methylated region to the 3' terminal of the neighboring region; and the region which has a distance to the target methylated region of 0 means the region adjacent to the target methylated region.

The details of the mechanism of action by which demethylation of the target methylated region is promoted when artificially generating RNA (a) or (b) in a living cell remain unclear, but it appears that RNA (a) or (b) can hybridize with a nucleic acid in the living cell (for example, a DNA chain or mRNA, etc. not present in the target methylated region); and it appears that RNA that can hybridize to the nucleic acid in question in the same region in which RNA (a) or (b) can hybridize can promote the demethylation of the target methylated region in the same way as the RNA (a) or (b).

RNA comprising the base sequence of RNA (a) with 1 or more bases substituted may be cited as RNA that can hybridize in the same region in which RNA (a) can hybridize. The number of bases substituted in the base sequence of RNA (a) is normally 1 to 10, preferably 1 to 5.

RNA comprising the base sequence of RNA (b) with 1 or more bases substituted may be cited as RNA that can hybridize in the same region in which RNA (b) can hybridize. The number of bases substituted in the base sequence of RNA (b) is normally 1 to 10, preferably 1 to 5.

In the present invention, the method to artificially generate RNA (a) or (b) in a living cell is not particularly limited. For example, RNA (a) or (b) may be artificially generated in a living cell by introducing into the living cell a recombinant vector that can express RNA (a) or (b).

The configuration of a recombinant vector that can express RNA (a) or (b) is not particularly limited, but generally has an expression cassette including a promoter positioned upstream from the DNA that codes for RNA (a) or (b). An expression cassette may include transcription control regions other than a promoter, for example, a terminator, enhancer, etc. A structure having a promoter upstream from the DNA that codes for RNA (a) or (b) and having a downstream terminator may be cited as an example of a specific structure of an expression cassette. The number of expression cassettes that a recombinant vector has is not particularly limited, and a recombinant vector may have 1 expression cassette or may have multiple expression cassettes that are the same or different.

DNA comprising a base sequence complimentary to the region including all or part of the target methylated region in the DNA chain where the target methylated region is present may be cited as DNA that codes for RNA (a). Moreover, DNA comprising a base sequence complimentary to the region including the region neighboring the target methylated region in the DNA chain where the target methylated region is present may be cited as DNA that codes for RNA (b). These DNA may be obtained, for example, by well known methods such as PCR and chemical synthesis, etc.

If capable of self-replication in the target living cell, the kind of vector used when constructing a recombinant vector is not particularly limited, and, for example, plasmid vectors, phage vectors, and virus vectors, etc. may be used. Plasmids derived from Escherichia coli (for example, pRSET, pBR322, pBR325, pUC118, pUC119, pUC18, pUC19), plasmids derived from Bacillus subtilis (for example, pUB110, pTP5), and plasmids derived from yeast (for example, YEp13, YEp24, YCp50) may be cited as examples of plasmid vectors. λ-phages (for example, Charon4A, Charon21A, EMBL3, EMBL4, λgt10, λgt11, λZAP) may be cited as examples of phage vectors. Retroviruses, animal viruses such as vaccinia virus, and insect viruses such as baculovirus may be cited as examples of virus vectors.

If capable of functioning in the target living cell, the kind of promoter comprising the expression cassette of the recombinant vector is not particularly limited. If the target living cell is an animal cell, for example Rα promoter, SV40 promoter, LTR (long terminal repeat) promoter, CMV promoter, human citomegalovirus early gene promoter, and CAG promoter, etc. may be used as promoters.

If capable of introducing a recombinant vector into the DNA of the target living cell, the method of introduction into the target living cell is not particularly limited, and for example, the electroporation, calcium phosphate, lipofection, and microinjection methods may be used.

The recombinant vector that can express RNA (a) or (b) may be used as a compositional element of a demethylation kit for demethylation of the target methylated region present in either the sense or antisense DNA chain in the genome DNA of the living cell. In addition to a recombinant vector that can express RNA (a) or (b), a demethylation kit may include the reagents and instruments necessary to introduce the recombinant vector into the living cell, and optional elements such as the reagents and instruments necessary to culture the living cells into which the recombinant vector has been introduced.

By introducing a recombinant vector into a living cell and artificially generating RNA (a) or (b) in the living cell, demethylation of the target methylated region is possible. Here, "demethylation of the target methylated region" means demethylation of the methylated cytosine present in the target methylated region, and "demethylation of the target methylated region" includes both complete demethylation and reduced methylation of the target methylated region. Moreover, methylated cytosine comprising a CpG sequence, methylated cytosine comprising a CpApG sequence, methylated cytosine comprising a CpTpG sequence, and methylated cytosine comprising a CpNpG sequence (N is A, T, C or G) may be cited as examples of methylated cytosine targeted for demethylation.

Whether or not the target methylated region has been demethylated may be confirmed by producing a methylation pattern using such methods as, for example, RLGS (restriction landmark genomic scanning), MS-PCR, Southern blotting, or CpG island microarray. For example, in RLGS the genome DNA is extracted from the cell, DNA fragments are produced by cutting with a methylation-sensitive restriction enzyme (for example, NotI, BssHII, Sa1I, etc.), labeling the terminal with a label substance (for example, ³²P), and then isolating the DNA fragments using one dimensional electrophoresis. Further, the DNA fragments are digested after electrophoresis using other restriction enzymes that differ from the methylation sensitive restriction enzymes, two-dimensional electrophoresis is applied, and spots are analyzed by autoradiography, etc. Then, whether the target methylated region has been demethylated is confirmed from the characteristic spot patterns of the cell. According to this method, the methylation and demethylation status of the region across several thousand positions of the genome DNA can be analyzed at one time.

By means of the present invention from among multiple methylated regions present in the genome DNA of a living cell the target region alone can be demethylated without influencing the other methylated regions, and therefore, the methylation pattern of the genome DNA of the living cell can be changed into the desired methylation pattern. There is the risk that anomalies of the genome DNA methylation patterns may have an effect on gene expression and genome stability, and may produce arrested development, development anomalies, and a variety diseases such as cancer and lifestyle-related diseases, but these may be prevented or cured by changing the methylation patterns to normal methylation patterns.

Living cells in which the methylation patterns have been made into the desired methylation patterns by demethylation of the target methylated region are useful, for example, as cells for implantation in regenerative medicine, and may be expected to be highly safe and efficient when implanting. In addition, tissues and organs produced (regenerated) from living cells in which the methylation patterns have been made into the desired methylation patterns by demethylation of the target methylated region are also useful as tissues and organs for implantation in regenerative medicine. It is preferable that stem cells are used as the living cells in the production (regeneration) of tissues and organs. The production (regeneration) of tissues and organs from living cells can be conducted by culturing living cells. Here, "culture" means common culture outside the body, and includes cell culture conducted at the level of cells removed from the tissue structure, tissue culture conducted at the level of tissues, and organ culture conducted while maintaining the organ structure intact, etc. For example, RPMI1640 medium, Eagle MEM medium, DMEM medium, Ham F12 medium, Ham F12K medium, or media that add bovine fetal serum, etc. to these media may be used as the culture medium, and the culture conditions are normally 37° C in the presence of 5% CO₂.

The cell nuclei of living cells in which the methylation patterns have been made into the desired methylation patterns by demethylation of the target methylated region are useful as the cell nuclei to implant in enucleated unfertilized eggs when producing cloned animals (non-human cloned animals). Normally, early stage blastocysts, cultured cells derived from early stage embryos, embryonic stem cells, and somatic cells, etc. are used as the living cells to provide the cell nuclei. Cloned animals can be produced by culturing eggs into which the nuclei of other cells have been implanted, and by transferring the developed transferable embryo into the uterus of a surrogate.

### EXAMPLES

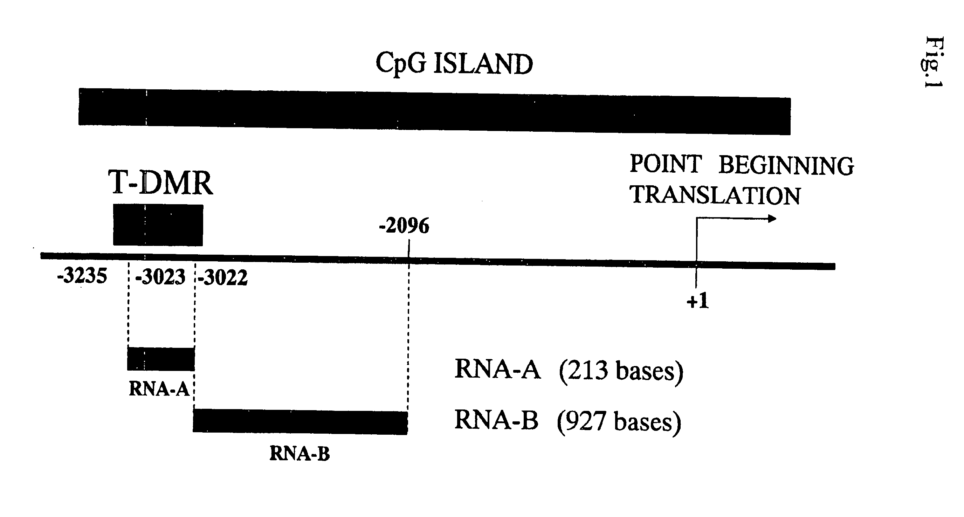

A tissue-dependently methylated region (T-DMR) is present upstream of the 5' terminal of the sphingosine kinase gene derived from rats (Imamura T., et al: Genomics 76: 117-125 (2001)) (refer to Fig. 1). This T-DMR is a in reduced methylated state in the brains of embryonic and mature rats, but is in a raised methylated state in other tissue (for example, the heart) of mature individuals (Imamura T., et al: Genomics 76: 117-125 (2001)). NRK cells, which are a fibroblast-like cell line in rats, are also in a raised methylated state.

RNA-A comprising a T-DMR base sequence, or RNA-B comprising a base sequence in the region neighboring the T-DMR were artificially generated in the appropriate cells by culturing NRK cells (RIKEN Cell Bank). The NRK cells were cultured at 37° C in the presence of 5% CO₂ using a medium in which bovine fetal serum at a final concentration of 10% was added to a DMEM medium.

The base sequence of the RNA-A is indicated in sequence No. 1, and the base sequence of the RNA-B is indicated in sequence No. 2. The RNA-A is equivalent to one part of the T-DMR, and the region of the corresponding genome DNA is -3235 to -3023 (refer to Fig. 1). In addition, RNA-B is equivalent to the region of the 3' terminal side of the T-DMR and a region comprising the region adjacent to the 3' terminal of the region in question, and the region of the corresponding genome DNA is -3022 to -2096 (refer to Fig. 1). Further, the positions of the various bases are the positions when A of the initial codon of the sphingosine kinase gene is made +1.

The recombinant vectors to express the RNA-A and RNA-B were constructed by inserting into the ApaI and HindIII restriction enzyme recognition members of pRc/CMV vectors (Invitrogen) double chained DNA that code for the base sequences of RNA-A or RNA-B amplified by PCR. The primers used in PCR related to the RNA-A were 5'-gggcccgcggccgcggacagaggagccattgtg-3', and 5'-aagcttcggggctggaacttctcacactctgcgggc-3'; and those related to RNA-B were 5'-gggcccagactcaaagaaaagcgagaccctgttcca-3' and 5'-aagcttacccacctctctccttgcatcgcttcttaa-3'.

The recombinant vectors were introduced into the cells by lipofection.

The methylation pattern of the T-DMR was analyzed by RLGS (restriction landmark genomic scanning). RLGS is a genome-wide method to analyze the methylation status in the CpG islands by combining methylation sensitive restriction enzymes and two-dimensional electrophoresis; and was conducted concretely as indicated below.

Genome DNA was cut using NotI as the methylation sensitive restriction enzyme, and cytosine and guanine labeled with a radioactive isotopic element (³²P) were incorporated in the cut locations. In this way, the locations that were not methylated will be labeled, and the locations that were methylated will be in an unlabelled state without cutting. After further reducing the mean chain length by cutting with PvuII, one-dimensional disc gel electrophoresis was conducted. After electrophoresis, another cut was made using PstI within the gel, and two-dimensional slab gel electrophoresis was conducted. The gel after electrophoresis was dried and used to expose X-ray film. In this way, the gene fragments having members not methylated were exposed as spots and made visible. If the members of gene fragments had been methylated, the spots in those parts would disappear, and therefore methylation could be detected.

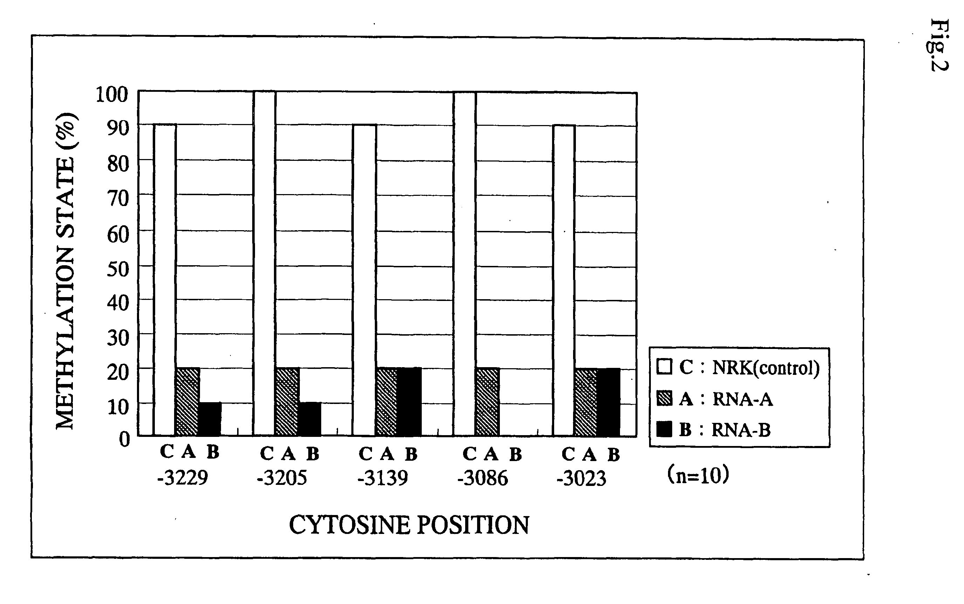

Indicated in Fig. 2 is the methylation state (%) of the T-DMR before and after artificially generating RNA-A or RNA-B in NRK cells. Fig. 2 indicates the cytosine methylation state of a CpG site (-3229 to -3023) which is representative of T-DMR, and the cytosine positions of the CpG sites are -3229, -3205, -3139, -3086, and -3023. The cytosine methylation states are indicated by the percentage (%)of NRK cells out of 10 NRK cells in which the cytosine of the specified location was methylated. In Fig. 2, column C (white bar) indicates the methylation state of T-DMR prior to artificially generating RNA-A or B in the NRK cells; column A (hash mark bar graph) indicates the methylation state of T-DMR after artificially generating RNA-A in the NRK cells; and column B (black bar graph) indicates the methylation state of T-DMR after artificially generating RNA-B in the NRK cells.

As indicated in Fig. 2, the methylation of cytosine in the various CpG sites was lowered (reduced methylation) by artificially generating RNA-A or RNA-B in the NRK cells.

### INDUSTRIAL APPLICABILITY

The present invention offers a demethylation method that from among multiple methylated regions present in the genome DNA of a living cell can demethylate a specified methylated region only without influencing the other methylated regions. According to the demethylation method of the present invention is possible to change the methylation pattern in the genome DNA of a living cell to the desired methylation pattern. By changing anomalies of the methylation patterns of genome DNA to normal methylation patterns, it is possible to prevent or cure arrested development, developmental anomalies, and a variety of diseases including cancer and lifestyle-related diseases, etc. that can be produced by anomalies of methylation patterns. In addition, living cells in which the methylation patterns have been changed to the desired methylation patterns by demethylation of the target methylated region are useful, for example as cells to implant for regeneration therapy, and may be expected to be highly safe and efficient when implanting. In addition, tissues and organs produced (regenerated) from living cells in which the methylation patterns have been made into the desired methylation patterns by demethylation of the target methylated region are also useful as tissues and organs for implantation in regenerative medicine. Further, the cell nuclei of living cells in which the methylation patterns have been made into the desired methylation patterns by demethylation of the target methylated region are useful as the cell nuclei to implant in enucleated unfertilized eggs when producing cloned animals (non-human cloned animals).

## Claims

1. An in-vitro method for demethylating a methylated cytosine residue or residues at CG sites in a target methylated region present in either sense or antisense DNA chain in genome DNA of a living cell, the method comprising a step of artificially generating in the living cell the following RNA (a) or (b),
(a) RNA in a base length of 100 to 2000 comprising a base sequence equivalent to the base sequence of a region comprising all or part of the target methylated region in the DNA chain where the target methylated region is present,
(b) RNA in a base length of 100 to 2000 comprising a base sequence equivalent to the base sequence of a region comprising a region neighboring the target methylated region in the DNA chain where the target methylated region is present, wherein the region neighboring the target region is positioned at the 5' terminal side or at the 3' terminal side of the target region and the distance between the neighboring region and the target region is a base length of 0 to 1000,
wherein the target methylated region resides in CpG islands having tissue - dependently methylated regions in the genome DNA.

2. The method according to claim 1, further comprising a step of introducing into the living cell a recombinant vector that can express the RNA (a) or (b).

3. A method for producing a living cell in which a methylated cytosine residue or residues at CG sites in a target methylated region present in either sense or antisense DNA chain in genome DNA is demethylated by the method according to claim 1 or 2, with the proviso that the living cell is not a human embryonic stem cell or germ cell.

4. A method for producing a tissue or organ by culturing a living cell produced by a method according to claim 3.

5. A method for producing a cell nucleus by isolation from a living cell produced by the method according to claim 3

## Patentansprüche

1. In-vitro-Verfahren zur Demethylierung eines methylierten Cytosin-Rests oder von methylierten Cytosin-Resten an CG-Stellen in einem methylierten Zielbereich, der entweder in der Sense- oder Antisense-DNA-Kette in der Genom-DNA einer lebenden Zelle vorhanden ist, wobei das Verfahren einen Schritt des künstlichen Herstellens der folgenden RNA (a) oder (b) in der lebenden Zelle umfasst:
(a) RNA in einer Basenlänge von 100 bis 2000, mit einer Basensequenz, die der Basensequenz eines Bereichs gleichwertig ist, der den gesamten oder einen Teil des methylierten Bereichs in der DNA-Kette umfasst, in dem der methylierte Zielbereich vorhanden ist,
(b) RNA in einer Basenlänge von 100 bis 2000, mit einer Basensequenz, die gleichwertig der Basensequenz eines Bereichs ist, der einen Bereich umfasst, der zum methylierten Zielbereich in der DNA-Kette benachbart ist, in dem der methylierte Zielbereich vorhanden ist, wobei der zum Zielbereich benachbarte Bereich auf der 5'-Terminus-Seite oder der 3'-Terminus-Seite des Zielbereichs positioniert ist, und der Abstand zwischen dem benachbarten Bereich und dem Zielbereich eine Basenlänge von 0 bis 1000 ist,
wobei der methylierte Zielbereich in CpG-Inseln mit gewebeabhängig methylierten Bereichen in der Genom-DNA liegt.

2. Verfahren nach Anspruch 1, darüber hinaus einen Schritt des Importierens eines rekombinanten Vektors in die lebende Zelle umfassend, der die RNA (a) oder (b) exprimieren kann.

3. Verfahren zum Erzeugen einer lebenden Zelle, in der ein methylierter Cytosin-Rest oder methylierte Cytosin-Reste an CG-Stellen in einem methylierten Zielbereich, der entweder in der Sense- oder Antisense-DNA-Kette in der Genom-DNA vorhanden ist, durch das Verfahren nach Anspruch 1 oder 2 demethyliert wird, unter der Voraussetzung, dass es sich bei der lebenden Zelle um keine humane embryonale Stammzelle oder Keimzelle handelt.

4. Verfahren zum Erzeugen eines Gewebes oder Organs durch Kultivieren einer lebenden Zelle, die durch das Verfahren nach Anspruch 3 erzeugt wurde.

5. Verfahren zum Erzeugen eines Zellkerns durch Isolieren aus einer lebenden Zelle, die durch das Verfahren nach Anspruch 3 erzeugt wurde.

## Revendications

1. Procédé in vitro de déméthylation d'un ou de plusieurs résidus cytosine méthylés au niveau de sites cytosine-guanine dans une région méthylée cible présente dans une chaîne d'ADN sens ou antisens d'ADN génomique d'une cellule vivante, le procédé comprenant une étape consistant à générer artificiellement dans la cellule vivante l'ARN (a) ou (b) suivant,
(a) ARN d'une longueur de base de 100 à 2000 comprenant une séquence de base équivalente à la séquence de base d'une région comprenant tout ou partie de la région méthylée cible de la chaîne ADN où la région méthylée cible est présente,
(b) ARN d'une longueur de base de 100 à 2000 comprenant une séquence de base équivalente à la séquence de base d'une région comprenant une région voisine à la région méthylée cible dans la chaîne d'ADN où la région méthylée cible est présente, la région voisine à la région cible étant positionnée au niveau de la terminaison 5' ou au niveau de la terminaison 3' de la région cible, et la distance entre la région voisine et la région cible étant une longueur de base de 0 à 1000,
dans lequel la région méthylée cible se trouve dans des îlots CpG ayant des régions méthylées dépendantes du tissu dans l'ADN génomique.

2. Procédé selon la revendication 1, comprenant en outre une étape consistant à introduire dans la cellule vivante un vecteur recombinant qui peut exprimer l'ARN (a) ou (b).

3. Procédé de production d'une cellule vivante, dans lequel un ou plusieurs résidus cytosine méthylés, au niveau des sites cytosine-guanine dans une région méthylée cible présente dans une chaîne d'ADN sens ou antisens d'ADN génomique, sont déméthylés par l'intermédiaire du procédé selon la revendication 1 ou 2, à condition que la cellule vivante ne soit pas une cellule souche ou une cellule germinale embryonnaire humaine.

4. Procédé de production d'un tissu ou d'un organe par la culture d'une cellule vivante produite par l'intermédiaire d'un procédé selon la revendication 3.

5. Procédé de production d'un noyau cellulaire par isolation d'une cellule vivante produite par l'intermédiaire du procédé selon la revendication 3.
